# NEUE EUROPÄISCHE PATENTSCHRIFT

(11) **EP 0 184 128 B2**
(45) Veröffentlichungstag und Bekanntmachung des Hinweises auf die Entscheidung über den Einspruch: **04.09.1996**
(45) Hinweis auf die Patenterteilung: 01.06.1988
(21) Anmeldenummer: 85115046.6
(22) Anmeldetag: 27.11.1985
(51) Int. Cl.: C07C 53/126, C07C 51/47, C08K 5/09

(54) **Verfahren zur Herstellung von Gemischen von neutralen und 2-basischen Blei(II)-Fettsäuresalzen durch Schmelzreaktion**
Process for the preparation of mixtures of neutral and dibasic lead (II) salts of fatty acids by the fusion process
Procédé de préparation de mélânges de sels neutres et dibasiques de plomb (II), d'acides gras selon le procédé de fusion

(30) Priorität: 05.12.1984 DE 3444259
(43) Veröffentlichungstag der Anmeldung: 11.06.1986
(73) Patentinhaber: NEYNABER CHEMIE GmbH, D-27608 Loxstedt (DE)
(72) Erfinder: Worschech, Kurt, Dr., D-2854 Loxstedt (DE); Wedl, Peter, D-2854 Loxstedt (DE); Fleischer, Erwin, D-2850 Bremerhaven-Spaden (DE); Löffelholz, Frido, D-2850 Bremerhaven-Surheide (DE)

(56) Entgegenhaltungen:
- DE-A- 1 768 450
- DE-A- 2 652 328
- DE-A- 2 922 378
- DE-B- 1 300 105
- DE-B- 1 794 429
- DE-B- 2 619 958
- GB-A- 684 155
- GB-A- 936 533
- US-A- 3 072 693

## Beschreibung

Bei der Herstellung von Formkörpern aller Art aus Hart-PVC werden in weitem Umfang Stabilisatoren auf Bleibasis eingesetzt. Stabilisatorsysteme dieser Art sind nicht nur hochwirksam, sondern auch preiswert. Sie gewährleisten darüber hinaus einen Lichtschutz bei der Lagerung. Rezepturen auf Basis von Bleistabilisatoren enthalten seit vielen Jahren üblicherweise sogenannte Primärstabilisatoren, nach ihrer Deckkraft auch Pigmentstabilisatoren genannt. Es handelt sich hier in der Regel um basische Bleisulfate.

Als weitere Stabilisatoren kommen organische Bleisalze, insbesondere Blei-Fettsäureseifen in Betracht. Wichtig sind insbesondere die Bleistearate, die entweder als 2basisches Bleistearat 2PbO·Pb(Fettsäurerest)₂ ― 51 %iges Pb-Stearat ― oder als neutrales Bleistearat Pb(Fettsäurerest)₂ ― 28%iges Pb-Stearat ― zum Einsatz kommen. Häufig werden auch Kombinationen dieser beiden Bleisalze eingesetzt.

Komplettiert wird das Stabilisatorsystem häufig durch Calciumseifen, insbesondere Calciumstearat. Diese Metallseife kann man allerdings auch dem Gleitmittel-bzw. Schmiersystem zurechnen, das gewöhnlich Paraffine, evtl. freie Fettsäure, andere Kohlenwasserstoffwachse, wie Polyethylenderivate und in einigen Fällen auch Fettsäureester beinhaltet.

Der wirtschaftliche Zwang, nach immer preiswerteren Rezepturen zu suchen, führte in letzter Zeit dazu, Pigmentstabilisatoren vom Typ des basischen Bleisulfats zu eliminieren. Auch die Weiterentwicklung auf der Seite der PVC-Verarbeitungsmaschinen hat dazu geführt, dass mit wesentlich schwächer stabilisierten PVC-Rezepturen heute problemlos extrudiert werden kann. Als Primärstabilisator rückt daher das basische Bleistearat 2PbO·Pb(Fettsäurerest)₂ immer stärker in den Mittelpunkt, wobei als rheologisch wirksame Rezepturpartner und Co-Stabilisatoren neutrales Bleistearat und Calciumstearat oder auch nur die neutrale Bleiseife eingesetzt werden. An den weiterhin verwendeten Komponenten der Stabilisator- und Gleitmittelsysteme für PVC hat sich demgegenüber wenig geändert.

Stoffgemische der hier betroffenen Art bieten den Anreiz, das gesamte Stabilisierungs- und Rheologiesystem als sogenanntes Schmelzcompound herzustellen. Entsprechende Vorschläge bringen beispielsweise die DE-C-1 544 697 und 1 569 190, die nicht-staubende Stabilisator-Gleitmittelkombinationen für Vinylchloridpolymerisate schildern und beispielsweise aus einem in der Schmelze vereinigten Gemisch von Gleitmitteln, Metallseifen einer langkettigen aliphatischen Carbonsäure und basischen Bleisalzen anorganischer oder organischer Säuren bestehen. Nach bisherigem Wissen ist es dabei allerdings schwierig bzw. unmöglich, definierte Mengen an 2basischem Bleistearat neben neutralem Bleistearat im Schmelzverfahren herzustellen. In den genannten Druckschriften des Standes der Technik wird dementsprechend vorgeschlagen, getrennt hergestelltes 2basisches Bleistearat in die Schmelze von Gleitmitteln und/oder neutralem Bleistearat einzutragen.

Gegenstand der DE-B-13 00 105 ist ein Verfahren zur Herstellung von Metallseifengemischen in der Schmelze. Diese Metallseifengemische enthalten 10 bis 40 Gew.-% Metallseifen, die unter 140 °C dünnflüssig schmelzen (Typ I), und 60 bis 70 Gew.-% Metallseifen, die nicht oder erst oberhalb 140 °C schmelzen (Typ II). Das Verfahren ist dadurch gekennzeichnet, daß man Metallseifen des "Typs II" durch Umsetzung von geradkettigen Fettsäuren mit 8 bis 24 C-Atomen mit Metalloxiden, Hydroxiden oder Metallsalzen flüchtiger Säuren bei einer Temperatur von 100 bis 150 °C in einer Schmelze von Metallseifen vom "Typ I" oder der Fettsäure herstellt und diese Fettsäure in Metallseifen vom "Typ I" überführt. Beispiele für Metallseifen vom "Typ I" sind normale Bleiseifen der genannten Fettsäuren, solche des "Typs II" mono- und dibasische Bleiseifen der Fettsäuren. Zur Erleichterung der Umsetzung oder zur Erniedrigung der Viskosität des Umsetzungsgemisches können organische Zusätze, die einen Schmelzpunkt von etwa 25 °C besitzen, mitverwendet werden. Als solche werden genannt: Phthalsäureester, Kohlenwasserstoffe, Fettalkohole, Wachsester, natürliche Wachse, Triglyceride und Fettsäureamide.

Die Erfindung geht von der Aufgabe aus, ein einfaches Verfahren zu entwickeln, bei dem vorherbestimmbare Mengen bzw. Mischungsverhältnisse von neutralen Bleiseifen und 2basischen Bleiseifen der angegebenen Art im Schmelzverfahren zugänglich werden. Mit dem neuen Verfahren will die Erfindung einen vereinfachten Weg zur Herstellung von Stabilisatorsystemen für halogenhaltige Polymerisate, insbesondere für PVC, schaffen, der die Herstellung und Vereinigung aller wesentlichen Komponenten in der Schmelze ermöglicht.

Die technische Lösung der erfindungsgemässen Lehre geht von der überraschenden Feststellung aus, dass sich basische Bleiseifen in der Schmelze aus neutralen Bleiseifen herstellen lassen, wenn in der Schmelze bestimmte Mischungskomponenten mitverwendet werden.

Gegenstand der Erfindung ist dementsprechend ein Verfahren zur Herstellung vorbestimmbarer Gemische von neutralen und 2-basischen Bleisalzen der Formel

2 PbO · Pb (Fettsäurerest)₂

durch Umsetzung von Blei(II)-oxid mit einer Schmelze der bleisalzbildenden Fettsäure, wobei
- zunächst neutrales Bleisalz durch Schmelzreaktion gebildet wird,
- der Schmelze Partialester des Glycerins und/oder Pentaerythrits und weiteres Blei(II)-oxid zugegeben und
- die Reaktion bis zu dessen Umwandlung in basisches Bleisalz durchgeführt wird.

Das erfindungsgemässe Verfahren arbeitet mit einer Schmelze des neutralen Bleifettsäuresalzes, der organische Verbindungen mit freien Hydroxylgruppen zugesetzt worden sind. In diese Schmelze wird feinteiliges Blei(II)-oxid eingetragen. In Gegenwart der erfindungsgemäss der Schmelze zugesetzten organischen Verbindungen mit freien Hydroxylgruppen erfolgt eine rasche Umsetzung des Bleioxids, die sich durch Verschwinden der gelben Oxidfarbe deutlich zu erkennen gibt. In Abwesenheit der erfindungsgemäss mitverwendeten organischen Hydroxylverbindungen verbleibt das eingetragene Bleioxid in Form gelber Partikel unreagiert suspendiert in der Schmelze.

Zur Katalysierung der Bildung des 2basischen Bleisalzes in der Schmelzreaktion werden erfindungsgemäss hochsiedende organische Hydroxylverbindungen mitverwendet. Geeignet sind entsprechende Verbindungen mit freien Hydroxylgruppen, die auch bei der üblichen abschliessenden Vakuumtrocknung des Reaktionsprodukts im Reaktionsgemisch verbleiben.

Es werden freie Hydroxylgruppen enthaltende organische Verbindungen mitverwendet, die mit dem Einsatzzweck als PVC-Stabilisator verträglich sind. Aus dem Bereich der vorbekannten Stabilisator- bzw. Gleitmittelkomponenten für PVC sind entsprechende organische Komponenten mit freien Hydroxylgruppen bekannt, die im Sinne der Erfindung zum Einsatz kommen können. Genannt seien hier Partialester des Glycerins und/oder des Pentaerythrits.

Geeignete Partialester des Pentaerythrits und ihre Verwendung in Stabilisator-Gleitmittel-Kombinationen für Formmassen auf Basis von Polyvinylchlorid, sind beispielsweise beschrieben in der DE-A-2 652 328. Geschildert sind hier Partialester des Pentaerythrits und/oder des Trimethylolpropans mit Fettsäuren von 8 bis 22 C-Atomen als Mischungskomponenten mit mehrbasischen Bleiverbindungen auf dem genannten Anwendungsgebiet. Die Partialester werden in an sich bekannter Weise durch partielle Veresterung der mehrwertigen Alkohole mit Fettsäuren einer Kettenlänge von 8 bis 22 C-Atomen hergestellt, wobei übliche Katalysatoren mitverwendet werden können. Geeignete Fettsäuren sind beispielsweise Capryl-, Caprin-, Laurin-, Myristin-, Palmitin-, Stearin- oder Behensäure. Besondere Bedeutung kann den Stearinsäureestern ― und im Zusammenhang damit auch den entsprechenden Bleisalzen der Stearinsäure ― zukommen.

Die organischen Hydroxylverbindungen können eine oder mehrere freie Hydroxylgruppen enthalten. Geeignet sind Partialester, beispielsweise des Pentaerythrits, die eine, zwei oder auch drei freie Hydroxylgruppen aufweisen. Entsprechendes gilt für Partialester des Glycerins. Die freien Hydroxylgruppen bzw. die entsprechenden organischen Verbindungen mit freien Hydroxylgruppen werden zur Auslösung und Förderung der Schmelzreaktion von Bleiglätte mit neutralem Bleisalz zu 2basischem Bleisalz lediglich in sehr geringen Konzentrationen benötigt. Katalytische Mengen der Hydroxylgruppen bzw. Hydroxylgruppen enthaltenden Verbindungen können ausreichen. Bevorzugt wird mit Mengen von wenigstens etwa 5 Gew.-% der Hydroxylgruppen enthaltenden Verbindungen ― bezogen auf die Bleisalze im Endprodukt ― gearbeitet. Es können aber auch beliebig grosse Mengen oder auch mengenmässig grosse Überschüsse der Hydroxylgruppen enthaltenden organischen Verbindung eingesetzt werden. In einer bevorzugten Ausführungsform der Erfindung wird die Menge der hydroxylgruppenhaltigen Verbindung durch den beabsichtigten Einsatzzweck als Stabilisator- und Gleitmittelkomposition bestimmt. Die mitverwendete hydroxylgruppenhaltige organische Komponente ist dabei eine im System erwünschte Komponente dieser Art, die in Teilbeträgen oder in der gesamten erforderlichen Menge bei der Bildung der Bleiseifen durch Schmelzreaktion zugegen sein kann.

Bei der Umsetzung von Bleiglätte mit der Schmelze einer neutralen Bleiseife in Gegenwart der hydroxylgruppenhaltigen Verbindungen entstehen Reaktionsschmelzen, deren Farbe von der Art des gewählten Zusatzes mit abhängig ist. Die hellsten Farben wurden bei Mitverwendung von Pentaerythritpartialestern beobachtet. Diesen Mischungskomponenten kann im Sinne der Erfindung dementsprechend besondere Bedeutung zukommen. Besonders wichtig kann das Arbeiten mit Pentaerythritdiestern und insbesondere entsprechenden Partialestern mit Stearinsäure zukommen.

Zur Herstellung vorbestimmbarer Gemische von neutralen und 2basischen Blei-Fettsäuresalzen wird bei dem erfindungsgemässen Verfahrens zunächst neutrales Bleisalz durch Schmelzreaktion aus Bleioxid und Fettsäure gebildet. Der Schmelze des neutralen Bleisalzes wird die organische Hydroxylgruppen enthaltende Verbindung zugesetzt, woraufhin in diese Schmelze weiteres Blei(II)-oxid in solchen Mengen zugegeben wird, dass nach Abschluss der Reaktion dieses in zweiter Stufe zugesetzten Blei(II)-oxids die vorbestimmte Menge von neutralem und basischem Bleisalz vorgibt. Allgemein gilt, dass in bevorzugten Ausführungsformen der Erfindung vorbestimmte Gewichtsverhältnisse von 2basischen Bleiseifen zu neutralen Bleiseifen im Bereich von 1:0,1 bis 1:10 hergestellt werden können. Insbesondere kann es wünschenswert sein, die entsprechenden Gewichtsverhältnise in den Bereich von 1:0,5 bis 1:3 einzustellen.

In einer weiteren Ausführungsform der Erfindung kann es wünschenswert sein, die Schmelzphase der Bleiseifen mit einer Schmelze entsprechender Calciumseifen zu vereinigen bzw. die Calciumseifen der entsprechenden Fettsäuren in der Schmelze der Bleiseifen herzustellen. Auf diese Weise gelingt die Darstellung komplexer Stabilisatorgemische in besonders einfacher Weise. Bevorzugt wird dabei die Salzbildung in den folgenden Reaktionsstufen vorgenommen: Zunächst wird in an sich bekannter Weise die neutrale Bleiseife durch Umsetzung einer Schmelze der Fettsäuren mit Bleioxid hergestellt. In dieser Schmelze der neutralen Bleiseife wird der gewünschte Anteil an entsprechender Calsiumseife gebildet. Hierzu kann von Anfang an mit entsprechend grossen Mengen der Fettsäure gearbeitet werden, oder aber es wird in die Schmelze der neutralen Bleiseife zusätzliche Fettsäure und eine geeignete Calciumverbindung, insbesondere Calciumoxid oder -hydroxid eingetragen. In der abschliessenden Reaktionsstufe erfolgt die Bildung der vorbestimmten Menge an 2basischer Bleiseife durch erneuten Eintrag von Bleiglätte. Die erfindungsgemäss mitverwendete organische Hydroxylverbindung kann der Schmelze von Anfang an oder zu einem beliebigen Zeitpunkt der abschliessenden Reaktionsstufe zur Bildung der 2basischen Bleiseife zugesetzt werden. Besonders bevorzugt kann es allerdings sein, die Bildung der neutralen Bleiseife und die Bildung der Calciumseife zunächst in Abwesenheit der hydroxylgruppenhaltigen organischen Verbindung vorzunehmen bzw. abzuschliessen und erst jetzt die freie Hydroxylgruppen enthaltende organische Komponente dem Reaktionsgemisch zur abschliessenden Bildung der 2basischen Bleiseifen zuzugeben.

Die erfindungsgemässe Schmelzreaktion kann gewünschtenfalls in Gegenwart weiterer, in der Schmelze gelöster und/oder suspendierter inerter Komponenten erfolgen, wie sie insbesondere in Stabilisator- und/oder Gleitmittelsystemen für halogenhaltige Polymerisate, insbesondere PVC, üblich sind. Insbesondere ist es im Rahmen der Erfindung aber auch möglich, den als Reaktionsprodukt anfallenden Schmelzsystemen die für den beabsichtigten Einsatzzweck zusätzlich erwünschten Komponenten beizumischen. Auf diese Weise werden im Sinne der Lehren des Standes der Technik nicht-staubende Stabilisator-Gleitmittelkombinationen erhalten, die granuliert oder in beliebig andere feinteilige Form überführt werden können.

Die im Schmelzverfahren hergestellten basischen und neutralen Seifen verhalten sich anwendungstechnisch ähnlich wie gefällte Seilen. Die Schmelzseifen haben darüber hinaus den Vorteil, dass sie umweltfreundlich herzustellen sind und die Herstellung von Stabilisatorsystemen für Hart-PVC in praktisch einem Verfahrensschritt auf der Basis einer Bleiverbindung ― nämlich Bleioxid ― ermöglichen. Die im erfindungsgemässen Verfahren hergestellten Seifengemische in Granulatform lassen sich mit PVC in üblicher Weise zu Trockengemischen aufarbeiten. Sie lassen sich bei üblichen Mischtemperaturen in Fluidmischern problemlos im Harz dispergieren und auf Extrudern, beispielsweise zu PVC-Rohren verarbeiten. Die Reststabilität, gemessen an den extrudierten Formteilen, ist ausreichend, um auch die Wiederverarbeitung von Anfahrmaterial zu gewährleisten.

### Beispiele

### Beispiel 1

In einem offenen Glasgefäss mit Rührer und Thermometer wurden 56,6 g (0,21 Mol) einer technischen C₁₆/C₁₈-Fettsäure (MG 270) und 45 g Hartparaffin (Smp. 100°C) aufgeschmolzen und auf 150°C erhitzt. Unter Rühren wurden dann im Verlauf von 2 Minuten 23,4 g (0,105 Mol) Bleiglätte in die Schmelze eingetragen, wobei die Reaktionstemperatur auf 150-160°C gehalten wurde. Nach weiteren 2 Minuten Nachrühren resultierte eine blanke gelbliche Schmelze. Es wurden nun 9 g Pentaerythritdistearat in die Schmelze eingerührt, anschliessend im Verlauf von 4 Minuten weitere 18 g (0,08 Mol) Bleiglätte zugegeben und die Schmelze bei 150°C 30 Minuten lang weitergerührt. Danach hatte sich das zugesetzte Bleioxid aufgelöst; die Reaktionsmischung lag ohne Bodenkörper als getrübte gelbliche Schmelze vor, die beim Abkühlen erstarrte (Smp. 103°C).

### Beispiel 2

Analog Beispiel 1 wurden 56,6g (0,21 Mol) technische C₁₆/C₁₈-Fettsäure (MG 270) und 45 g Hartparaffin (Smp. 100°C) auf 150°C erhitzt und mit 23,4 g (0,105 Mol) Bleiglätte versetzt. Nach dem Auflösen des Bleioxids wurden 9 g technisches Glycerindistearat und weitere 18g (0,08 Mol) Bleiglätte eingetragen. Die Schmelze wurde bei 150°C weitergerührt, wobei sich die zugesetzte Bleiglätte im Verlauf von 40 Minuten auflöste. Das Reaktionsgemisch lag danach ohne Bodenkörper als fast blanke, rötlich gefärbte Schmelze vor, die beim Abkühlen erstarrte (Smp. 100°C).

### Beispiel 3

In einem offenen Glasgefäss mit Rührer und Thermometer wurden 69,1 g (0,26 Mol) technische C₁₆/C₁₈-Fettsäure (MG 270) und 38,3g Hartparaffin (Smp. 100°C) auf 150°C erhitzt und unter Rühren mit 20g (0,09 Mol) Bleiglätte und 2,9 g (0,04 Mol) Calciumhydroxid versetzt. Nachdem sich Bleioxid und Calciumhydroxid aufgelöst hatten, wurden 7,6 g Pentaerythritdistearat und danach 15,1 g (0,068 Mol) Bleiglätte in die Schmelze eingetragen. Die Schmelze wurde bei 150°C weitergerührt, bis sich nach 30 Minuten das zugesetzte Bleioxid aufgelöst hatte. Die Reaktionsmischung lag ohne Bodenkörper als getrübte gelbliche Schmelze vor, die bei Abkühlen erstarrte (Smp. 101°C).

### Beispiel 4

Analog Beispiel 3 wurden 60,7g (0,23 Mol) technische C₁₆/C₁₈-Fettsäure (MG 270) und 42,8g Hartparaffin (Smp. 100°C) auf 150°C erhitzt und nacheinander 22,3 g (0,10 Mol) Bleiglätte und 1 g (0,014 Mol) Calciumhydroxid unter Rühren in die Schmelze eingetragen. Nachdem sich Bleioxid und Calciumhydroxid aufgelöst hatten, wurden 8,5g Pentaerythritdistearat und danach 17,0 g (0,076 Mol) Bleiglätte zugegeben und die Schmelze so lange weitergerührt, bis die Schmelze nach 40 Minuten ohne Bodenkörper vorlag. Die getrübte gebliche Reaktionsmischung erstarrte beim Abkühlen (Smp. 101°C).

## Patentansprüche

1. Verfahren zur Herstellung vorbestimmbarer Gemische von neutralen und 2-basischen Bleisalzen der Formel
2 PbO · Pb (Fettsäurerest)₂
durch Umsetzung von Blei(II)-oxid mit einer Schmelze der bleisalzbildenden Fettsäure, wobei
- zunächst neutrales Bleisalz durch Schmelzreaktion gebildet wird,
- der Schmelze Partialester des Glycerins und/oder Pentaerythrits und weiteres Blei(II)-oxid zugegeben und
- die Reaktion bis zu dessen Umwandlung in basisches Bleisalz durchgeführt wird.

2. Verfahren nach Anspruch 1, dadurch gekennzeichnet, daß die Partialester des Glycerins und/oder das Pentaerythrits wenigstens in katalytischen Mengen und vorzugsweise in Mengen von wenigstens etwa 5 Gew.-% - bezogen auf Bleisalz im Endprodukt - eingesetzt werden.

3. Verfahren nach Ansprüchen 1 und 2, dadurch gekennzeichnet, daß Bleisalze der Stearinsäure hergestellt werden.

4. Verfahren nach Ansprüchen 1 bis 3, dadurch gekennzeichnet, daß in der Schmelzphase zusätzlich Calciumsalze der Fettsäuren gebildet werden, wobei bevorzugt die Salzbildung in den folgenden Reaktionsstufen vorgenommen wird: Bildung der neutralen Bleisalze, Bildung der Calciumsalze und abschließend Bildung der 2-basischen Bleisalze, wobei die Partialester des Glycerins und/oder des Pentaerythrits von Anfang an oder zu einem beliebigen Zeitpunkt von der Bildung der 2-basischen Bleisalze, insbesondere aber erst am abschließenden Reaktionsschritt dieser Bildung der 2-basischen Bleisalze zugesetzt werden.

5. Verfahren nach Ansprüchen 1 bis 4, dadurch gekennzeichnet, daß Pentaerythritdiester insbesondere Pentaerythritdistearat eingesetzt werden.

6. Verfahren nach Ansprüchen 1 bis 5, dadurch gekennzeichnet, daß man die Schmelzreaktion in Gegenwart weiterer in der Schmelze gelöster und/oder suspendierter inerter Komponenten von PVC-Stabilisator- und/oder Gleitmittelsystemen durchführt.

7. Verfahren nach Ansprüchen 1 bis 6, dadurch gekennzeichnet, daß Gewichtsverhältnisse von 2-basischen Bleisalzen zu neutralen Bleisalzen im Bereich von 1:0,1 bis 1:10 und insbesondere von 1:0,5 bis 1:3 hergestellt werden.

## Claims

1. A process for the production of predeterminable mixtures of neutral and dibasic lead salts of the formula
2PbO · Pb(fatty acid radical)₂
by reaction of lead(II) oxide with a melt of the fatty acid forming the lead salt, wherein
- firstly, neutral lead salt is formed by fusion reaction,
- partial esters of glycerol and/or of pentaerythritol and further lead(II) oxide are added to the melt and
- the reaction is carried out until the lead(II) oxide has been converted into basic lead salt.

2. A process as claimed in Claim 1, characterized in that the partial esters of glycerol and/or of pentaerythritol are used in at least catalytic quantities and preferably in quantities of at least about 5 wt.%, based on the lead salt in the final product.

3. A process as claimed in Claims 1 and 2, characterized in that lead salts of stearic acid are produced.

4. A process as claimed in Claims 1 to 3, characterized in that calcium salts of the fatty acids in addition are formed in the melt phase, the salt formation preferably being carried out in the following reaction stages: formation of the neutral lead salts, formation of the calcium salts and finally formation of the dibasic lead salts, the partial esters of glycerol and/or of pentaerythritol being added from the outset or at any desired time during the formation of the dibasic lead salts, but in particular only in the final reaction stage of this formation of the dibasic lead salts.

5. A process as claimed in Claims 1 to 4, characterized in the pentaerythritol diesters, in particular pentaerythritol distearate, are used.

6. A process as claimed in Claims 1 to 5, characterized in that the fusion reaction is carried out in the presence of other inert components of PVC stabilizer and/or lubricant systems, dissolved and/or suspended in the melt.

7. A process as claimed in Claims 1 to 6, characterized in that weight ratios of dibasic lead salts to neutral lead salts in the range of 1:0.1 to 1:10 and particular of 1:0.5 to 1:3 are produced.

## Revendications

1. Procédé de préparation de mélanges prédéfinissables de sels de plomb neutres et dibasiques de formule :
2 PbO . Pb (radical d'acide gras)₂
par réaction d'oxyde de plomb (II) avec une masse fondue constituée par l'acide gras (formant des sels de plomb, dans laquelle :
- le sel de plomb neutre est d'abord formé par réaction de fusion,
- des esters partiels du glycérol et/ou du pentaérythritol et de l'oxyde de plomb (II) additionnel sont ajoutés et,
- la réaction est réalisée jusqu'à sa transformation en sel de plomb basique.

2. Procédé selon la revendication 1, caractérisé en ce que les esters partiels du glycérol et/ou du pentaérythritol sont mis en oeuvre au moins en quantités catalytiques et de préférence en quantités d'au moins environ 5 % en poids, par rapport au sel de plomb dans le produit final.

3. Procédé selon les revendications 1 et 2, caractérisé en ce que sont préparés des sels de plomb de l'acide stéarique.

4. Procédé selon les revendications 1 à 3, caractérisé en ce que dans la phase de fusion sont formés additionnellement des sels de calcium des acides gras, tandis que de préférence est perceptible la formation de sels dans les étapes de réaction suivantes : formation des sels de plomb neutres, formation des sels de calcium et ensuite formation des sels de plomb dibasiques, les esters partiels du glycérol et/ou du pentaérythritol étant ajoutés à partir du début ou à un moment quelconque de la formation des sels de plomb dibasiques, mais en particulier dès l'achèvement de la phase de réaction de cette formation des sels de plomb dibasiques.

5. Procédé selon les revendications 1 à 4, caractérisé en ce que des diesters de pentaérythritol en particulier du distéarate de pentaérythritol sont mis en oeuvre.

6. Procédé selon les revendications 1 à 5, caractérisé en ce qu'on réalise la réaction de fusion en présence d'autres composants inertes de systèmes stabilisants de PVC ou de systèmes lubrifiants, composants dissous et/ou en suspension dans la masse fondue.

7. Procédé selon les revendications 1 à 6, caractérisé en ce qu'on produit des rapports de poids des sels de plomb dibasiques aux sels de plombs neutres dans la zone de 1:0,1 à 1:10 et en particulier de 1:0,5 à 1:3.
